(19) Europäisches Patentamt
European Patent Office
Office européen des brevets

(11) Veröffentlichungsnummer: **0 205 049**
**A1**

(12) # EUROPÄISCHE PATENTANMELDUNG

(21) Anmeldenummer: **86107329.4**

(51) Int. Cl.4: **C07C 127/22** , A01N 47/34

(22) Anmeldetag: **30.05.86**

(30) Priorität: **31.05.85 DE 3519458**

(43) Veröffentlichungstag der Anmeldung:
**17.12.86 Patentblatt 86/51**

(84) Benannte Vertragsstaaten:
**AT BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Neubauer, Hans-Juergen, Dr.**
**B 4,2**
**D-6800 Mannheim 1(DE)**
Erfinder: **Hofmeister, Peter, Dr.**
**Bernard-Humblot-Strasse 12**
**D-6730 Neustadt(DE)**
Erfinder: **Liese, Thomas, Dr.**
**H 1, 16/17**
**D-6800 Mannheim 1(DE)**
Erfinder: **Adolphi, Heinrich,Dr.**
**Kalmitweg 11**
**D-6703 Limburgerhof(DE)**

(54) **N-Benzoyl-,N'-(1,1-difluor-phenylmethyl)phenylharnstoffe, Verfahren zur ihrer Herstellung und Verwendung zur Bekämpfung von Schädlingen.**

(57) N-Benzoyl-,N'-(1,1-difluor-phenylmethyl)-phenylharnstoffe der Formeln Ia bzw. Ib.

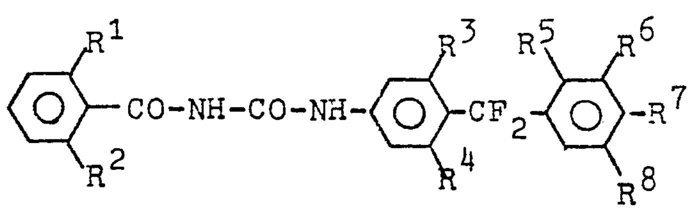

(Ia)

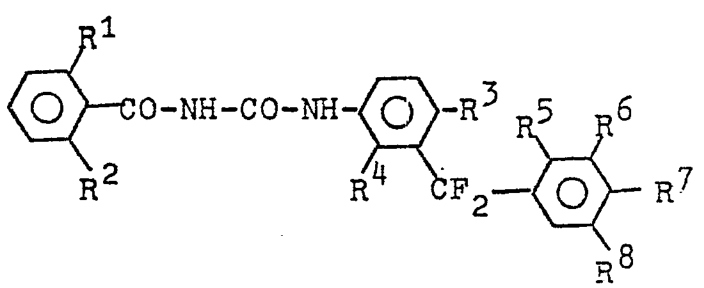

(Ib),

in denen bedeutet

$R^1$ Fluor, Chlor, Brom, Methyl oder Methoxy

$R^2$ Fluor, Chlor oder Wasserstoff

$R^3$ und $R^4$ gleich oder verschiedene Substituenten, nämlich Wasserstoff, Chlor, Brom, Fluor oder Trifluormethyl

$R^5$ bis $R^8$ Wasserstoff oder mindestens einen Sub-

EP 0 205 049 A1

stituenten, nämlich Fluor, Chlor, Brom, Alkyl mit 1 bis 4 C-Atomen, das mit Fluor und/oder Chlor substituiert sein kann, Alkoxy mit 1 bis 4 C-Atomen, das mit Fluor und/oder Chlor substituiert ist, Verfahren zu ihrer Herstellung und ihre Verwendung als Schädlingsbekämpfungsmittel.

# N-Benzoyl-,N'-(1,1-difluor-phenylmethyl)-phenylharnstoffe, Verfahren zu ihrer Herstellung und Verwendung zur Bekämpfung von Schädlingen

Die Erfindung betrifft N-Benzoyl-,N'-(1,1-difluorphenylmethyl)phenylharnstoffe, Verfahren zu ihrer Herstellung und Schädlingsbekämpfungsmittel, die diese Verbindungen als Wirkstoffe enthalten.

Verbindungen vom Typus der N-Benzoyl-,N'-phenylharnstoffe können als Insektizide verwendet werden (J. Agr. Food Chem. 21, 348 (1973); vgl. auch DE-OS 21 23 236 bzw. US-PS 4,399,152).

Es wurde gefunden, daß N-Benzoyl-,N'-(1,1-difluor-phenylmethyl)-phenylharnstoffe der allgemeinen Formel Ia bzw. Ib

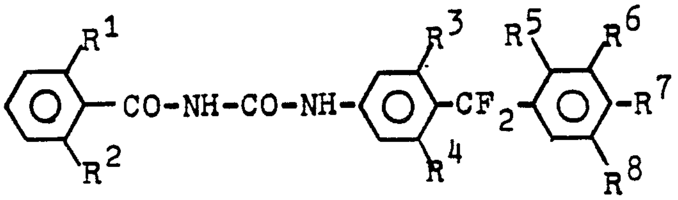

(Ia)

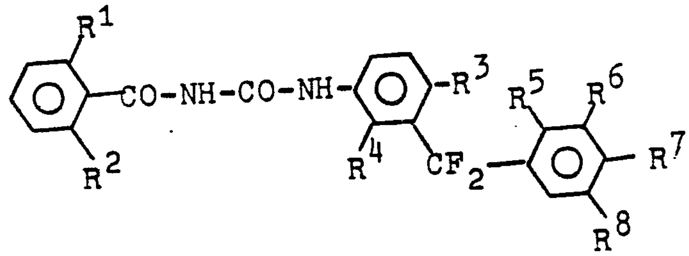

(Ib),

in denen bedeutet

$R^1$ Fluor, Chlor, Brom, Methyl oder Methoxy

$R^2$ Fluor, Chlor oder Wasserstoff

$R^3$ und $R^4$ gleiche oder verschiedene Substituenten, nämlich Wasserstoff, Chlor, Brom, Fluor oder Trifluormethyl

$R^5$ bis $R^8$ Wasserstoff oder mindestens einen Substituenten, nämlich Fluor, Chlor, Brom, Alkyl mit 1 bis 4 C-Atomen, das mit Fluor und/oder Chlor substituiert sein kann, Alkoxy mit 1 bis 4 C-Atomen, das mit Fluor und/oder Chlor substituiert ist,

insektizid und akarizid sehr gut wirksam und bekannten Wirkstoffen ähnlicher Struktur bzw. gleicher Wirkungsrichtung überlegen sind.

(Wie der Vergleich der Formel Ia mit Ib zeigt, kann die Verknüpfungsstelle des (1.1-Difluor-phenylmethyl)-phenylrestes mit dem Harnstoffrest sich in meta-oder para-Position zur Difluormethylengruppe befinden.)

Bevorzugt sind Verbindungen, bei denen $R^1$ = $R^2$ = F oder $R^1$ = F und $R^2$ = Cl oder $R^1$ = Cl und $R^2$ = H oder $R^1$ = $R^2$ = Cl ist.

Weiter bevorzugt sind Verbindungen, bei denen $R^3$ und/oder $R^4$ Chlor oder Fluor ist.

Besonders wirksam und auch relativ günstig herstellbar sind Verbindungen, bei denen nur einer der Reste $R^5$ bis $R^8$, und zwar insbesondere der Rest $R^7$von Wasserstoff verschieden ist und in diesem Falle aus der Gruppe F, Cl, Br, $OCHF_2$, $OCF_3$, $OC_2HF_4$, $CF_3$ gewählt ist.

Man erhält die erfindungsgemäßen N-Benzoyl-,N'-(1,1-difluor-phenylmethyl)phenylharnstoffe auf jede Weise, die zur Herstellung der entsprechenden bekannten Verbindungen verwendet werden kann. Beispielsweise kann man eine entsprechenden Verbindung der Formel II

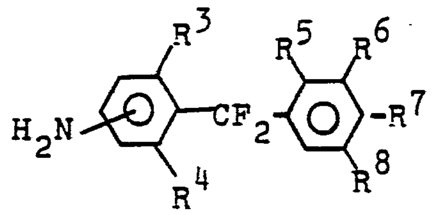

(II),

mit einem entsprechenden Benzoylisocyanat der Formel III

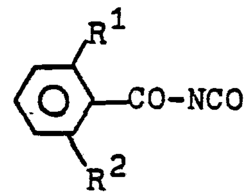

(III),

oder, falls die benötigten Ausgangsstoffe II bzw. III nicht verfügbar sind oder zu teuer einstehen, eine entsprechende Verbindung der Formel IV

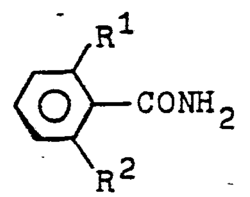

(IV),

mit einer entsprechenden Verbindung der Formel V

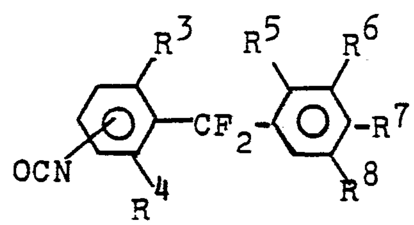

(V),

umsetzen.

Umsetzungen dieser Art, geeignete Bedingungen, Lösungsmittel und andere Hinweise sind z. B. beschrieben in der DE-AS 21 23 236.

Die Umsetzungen verlaufen teilweise quantitativ und liefern meist von vornherein einheitliche Produkte, gegebenenfalls können die üblichen Methoden zur Reinigung, z. B. Umkristallisieren, angewendet werden. Zu ihrer Charakterisierung dienen Elementaranalyse, Schmelzpunkt, IR-und NMR-Spektrum.

Zur Durchführung des erstgenannten Verfahrens wird zweckmäßigerweise das substituierte Anilin II zusammen mit einem Lösungs-oder Verdünnungsmittel vorgelegt und eine stöchiometrische Menge an Isocyanat III zugegeben. Die Umsetzung dauert in der Regel nicht mehr als 2 Stunden. Das andere Verfahren benötigt i. a. eine Reaktionsdauer von 2 bis 6 Stunden, wobei der Zusatz eines Katalysators wie Triethylamin oder Dibutylzinndiacetat vorteilhaft sein kann.

Die Benzoylisocyanate III sind bekannte Verbindungen; man kann sie z. B. nach den Vorschriften in J. Org. Chem. 28 1805 -1811 (1963) oder J. Agr. Food Chem. 21, 348 (1973) erhalten.

Die als Ausgangsstoffe verwendeten (1,1-Difluor-phenylmethyl)-phenylamine II und die entsprechenden Isocyanate sind neu. Sie können mit üblichen Methoden aus (1,1-Difluor-phenylmethyl)-nitrobenzolen durch Reduktion hergestellt werden

(Houben-Weyl, Methoden der organischen Chemie, XI/1, S. 360 ff. (1975)); die ihrerseits durch Umsetzung geeigneter Nitrobenzophenone mit Schwefeltetrafluorid (z. B. G. A. Baswell, W. C. Ripka, R. M. Scribner und C. W. Tullock, Organic Reactions, 21, 1 ff. (1974)) oder durch Behandlung von (1,1-Dichlor-phenylmethyl)-nitrobenzolen mit Alkalifluoriden (vgl. M. Hudlicky, Chemistry of Organic Fluorine Compounds, S. 91 ff. (1976)) erhältlich sind. Die (1,1-Difluor-phenylmethyl)-phenylamine II können in die entsprechenden (1,1-Difluor-phenyl methyl)-phenylisocyanate V überführt werden (vgl. Weygand-Hilgetag, Organisch-Chemische Experimentierkunst, 1970; DE-OS 25 38 178).

Herstellungsbeispiele

A. 26,8 g 4-Brom-2'-chlor-4'-nitro-benzophenon werden zusammen mit 80 g Schwefeltetrafluorid und einer katalytischen Menge Fluorwasserstoff in einem Hasteloy-C-Autoklaven 24 h bei 120 °C gerührt. Nach Abkühlen und Entfernen der flüchtigen Bestandteile wird in Methylenchlorid aufgenommen, mit Wasser gewaschen und die organische Phase getrocknet. Nach Abziehen des Lösungsmittels und Umkristallisation aus Hexan erhält man 15,8 g (56 % der rechnerisch möglichen Ausbeute) 4-Brom-2'-chlor-4'-nitro-diphenyldifluormethan vom Schmelzpunkt 73 °C.

B. 7,1 g des nach A erhaltenen Produkts werden in 100 ml abs. THF mit 1 g Raney-Nickel bei Raumtemperatur und 1 bar $H_2$-Druck hydriert. Man trennt vom Katalysator ab, engt ein und erhält so 7,0 g (97 % der rechnerisch möglichen Ausbeute) 4-Amino-4'-brom-2-chlor-diphenyldifluormethan als viskoses Öl.

C. Zu einer Lösung von 3,4-g des nach B erhaltenen Produkts in 50 ml abs. THF tropft man bei Raumtemperatur 2,1 g 2,6-Difluorbenzoylisocyanat. Anschließend wird 2 h bei 45 °C gerührt, mit 100 ml Pentan versetzt und abgesaugt. Nach Trocknen erhält man 4,1 g (78 % der rechnerisch möglichen Ausbeute) N-[3-chlor-4-(1,1-difluor-1-(4-Bromphenyl)-methyl)-phenyl]-,N'-(2,6-difluorbenzoyl)-harnstoff vom Schmelzpunkt 218 - 220 °C (Verbindung 4 der nachfolgenden Tabelle).

Die übrigen Wirkstoffe der Tabelle werden aus entsprechenden Vor-bzw. Zwischenprodukten erhalten. Soweit sie nicht hergestellt wurden ist anzunehmen, daß sie aufgrund ihrer strukturellen Ähnlichkeit eine vergleichbare Wirkung haben.

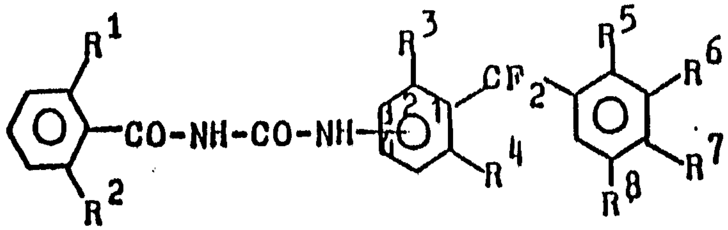

(Ia, b)

| Nr. | $R^1$ | $R^2$ | $R^3$ | $R^4$ | $R^5$ | $R^6$ | $R^7$ | $R^8$ | Substitution entspr. Formel | Fp. °C |
|---|---|---|---|---|---|---|---|---|---|---|
| 1 | F | F | H | H | H | H | Cl | H | Ia | |
| 2 | F | F | Cl | H | H | H | Cl | H | Ia | 207 - 210 |
| 3 | F | F | H | H | H | H | Br | H | Ia | |
| 4 | F | F | Cl | H | H | H | Br | H | Ia | 218 - 220 |
| 5 | F | F | Cl | H | H | H | F | H | Ia | |
| 6 | F | F | H | H | H | H | Cl | H | Ib | 171-173 |
| 7 | F | F | Cl | H | H | H | $OCF_3$ | H | Ia | |
| 8 | F | F | Cl | H | Cl | H | Cl | H | Ia | 196 - 199 |
| 9 | F | F | Cl | H | Cl | H | H | Cl | Ia | |
| 10 | F | F | Cl | H | F | H | F | H | Ia | 214 - 216 |
| 11 | F | F | Cl | H | Br | H | Br | H | Ia | |
| 12 | F | F | Cl | H | Cl | Cl | Cl | H | Ia | 218 - 221 |
| 13 | F | F | H | H | Cl | Cl | Cl | H | Ia | |
| 14 | F | F | H | H | Cl | Br | Cl | H | Ia | |
| 15 | F | F | Cl | H | H | H | F | F | Ia | |
| 16 | F | F | Cl | Cl | H | H | Cl | H | Ia | |

| Nr. | $R^1$ | $R^2$ | $R^3$ | $R^4$ | $R^5$ | $R^6$ | $R^7$ | $R^8$ | Substitution entspr. Formel | Fp. °C |
|---|---|---|---|---|---|---|---|---|---|---|
| 17 | F | F | Cl | Cl | H | H | Br | H | Ia | |
| 18 | F | F | Br | H | H | H | Cl | H | Ia | |
| 19 | F | F | Br | H | H | H | Br | H | Ia | |
| 20 | F | F | Cl | Cl | H | H | $OCF_3$ | H | Ia | |
| 21 | F | F | F | H | H | H | Cl | H | Ia | |
| 22 | F | F | $CF_3$ | H | H | H | Cl | H | Ia | |
| 23 | F | F | Cl | H | H | H | $CH_3$ | H | Ia | |
| 24 | Cl | H | H | H | H | H | Cl | H | Ia | |
| 25 | Cl | H | H | H | H | H | Cl | H | Ib | 110 - 112 |
| 26 | Cl | H | Cl | H | H | H | Cl | H | Ia | 155 - 158 |
| 27 | Cl | H | H | H | H | H | Br | H | Ia | |
| 28 | Cl | H | Cl | H | H | H | Br | H | Ia | 159 - 160 |
| 29 | Cl | H | Cl | H | H | H | F | H | Ia | |
| 30 | Cl | H | H | H | H | H | F | H | Ib | |
| 31 | Cl | H | Cl | H | H | H | $OCF_3$ | H | Ia | |
| 32 | Cl | H | Cl | H | Cl | H | Cl | H | Ia | 184 - 188 |
| 33 | Cl | H | Cl | H | Cl | H | H | Cl | Ia | |
| 34 | Cl | H | Cl | H | Br | H | Cl | H | Ia | 183 - 186 |

| Nr. | $R^1$ | $R^2$ | $R^3$ | $R^4$ | $R^5$ | $R^6$ | $R^7$ | $R^8$ | Substitution entspr. Formel | Fp. °C |
|---|---|---|---|---|---|---|---|---|---|---|
| 35 | Cl | H | Cl | H | Br | H | Br | H | Ia | |
| 36 | Cl | H | Cl | H | Cl | Cl | Cl | H | Ia | 190 - 194 |
| 37 | Cl | H | Cl | H | H | H | F | F | Ia | |
| 38 | Cl | H | Cl | Cl | H | H | Cl | H | Ia | |
| 39 | Cl | H | Cl | Cl | H | H | Br | H | Ia | |
| 40 | Cl | H | Cl | Cl | H | H | $OCF_3$ | H | Ia | |
| 41 | Cl | H | Br | H | H | H | Cl | H | Ia | |
| 42 | Cl | H | Br | H | H | H | Br | H | Ia | |
| 43 | Cl | H | F | H | H | H | Cl | H | Ia | |
| 44 | Cl | H | $CF_3$ | H | H | H | Cl | H | Ia | |
| 45 | Cl | H | Cl | H | H | H | $CH_3$ | H | Ia | |
| 46 | Cl | Cl | H | H | H | H | Cl | H | Ia | |
| 47 | Cl | Cl | Cl | H | H | H | Cl | H | Ia | |
| 48 | Cl | Cl | H | H | H | H | Cl | H | Ib | 125 - 130 |
| 49 | Cl | Cl | Cl | H | H | H | Br | H | Ia | |
| 50 | Cl | Cl | Cl | H | H | H | F | H | Ia | |
| 51 | Cl | Cl | H | H | H | H | F | H | Ib | |

| Nr. | $R^1$ | $R^2$ | $R^3$ | $R^4$ | $R^5$ | $R^6$ | $R^7$ | $R^8$ | Substitution entspr. Formel | Fp. °C |
|---|---|---|---|---|---|---|---|---|---|---|
| 52 | Cl | Cl | Cl | H | H | H | $OCF_3$ | H | Ia | |
| 53 | Cl | Cl | Cl | H | Cl | H | Cl | H | Ia | 172 - 174 |
| 54 | Cl | Cl | Cl | H | Cl | H | H | Cl | Ia | |
| 55 | Cl | Cl | Cl | H | F | H | F | H | Ia | 175 - 177 |
| 56 | Cl | Cl | Cl | H | Br | H | Br | H | Ia | |
| 57 | Cl | Cl | Cl | H | Cl | Cl | Cl | H | Ia | 204 - 206 |
| 58 | Cl | Cl | Cl | H | H | H | F | F | Ia | |
| 59 | CCl | Cl | Cl | Cl | H | H | Cl | H | Ia | |
| 60 | Cl | Cl | Cl | Cl | H | H | Br | H | Ia | |
| 61 | Cl | Cl | Cl | Cl | H | H | $OCF_3$ | H | Ia | |
| 62 | Cl | Cl | Br | H | H | H | Cl | H | Ia | |
| 63 | Cl | Cl | Br | H | H | H | Br | H | Ia | |
| 64 | Cl | Cl | F | H | H | H | Cl | H | Ia | |
| 65 | Cl | Cl | $CF_3$ | H | H | H | Cl | H | Ia | |
| 66 | Cl | Cl | Cl | H | H | H | $CH_3$ | H | Ia | |
| 67 | Cl | F | Cl | H | H | H | Cl | H | Ia | |
| 68 | Cl | F | Cl | H | Cl | H | Cl | H | Ia | |
| 69 | Cl | F | Cl | Cl | H | H | Cl | H | Ia | |

| Nr. | $R^1$ | $R^2$ | $R^3$ | $R^4$ | $R^5$ | $R^6$ | $R^7$ | $R^8$ | Substitution entspr. Formel | Fp. °C |
|---|---|---|---|---|---|---|---|---|---|---|
| 70 | Br | H | Cl | H | H | H | Cl | H | Ia | |
| 71 | Br | H | Cl | Cl | H | H | Cl | H | " | |
| 72 | $CH_3$ | H | Cl | H | H | H | Cl | H | " | |
| 73 | $CH_3$ | H | Cl | Cl | H | H | Cl | H | " | |
| 74 | $CH_3$ | H | Cl | H | H | H | Br | H | " | |
| 75 | $CH_3$ | H | Cl | H | H | H | F | H | " | |
| 76 | $CH_3$ | H | Cl | H | H | H | $OCF_3$ | H | " | |
| 77 | $CH_3$ | H | Cl | Cl | H | H | $OCF_3$ | H | " | |
| 78 | $CH_3$ | H | Cl | H | Cl | H | Cl | H | " | |
| 79 | $CH_3$ | H | Cl | H | Cl | Cl | Cl | H | " | |
| 80 | $CH_3$ | H | Br | H | H | H | Cl | H | " | |
| 81 | $CH_3$ | H | Br | H | H | H | Br | H | " | |
| 82 | $CH_3$ | H | F | H | H | H | Cl | H | " | |
| 83 | $CH_3$ | Cl | Cl | H | H | H | Cl | H | " | |
| 84 | $CH_3$ | Cl | Cl | H | H | H | Br | H | " | |
| 85 | $CH_3$ | Cl | Cl | Cl | H | H | Cl | H | " | |
| 86 | $CH_3$ | F | Cl | H | H | H | Cl | H | " | |
| 87 | $CH_3$ | F | Cl | H | H | H | Br | H | " | |
| 88 | $OCH_3$ | H | Cl | H | H | H | Cl | H | " | |
| 89 | $OCH_3$ | H | Cl | H | H | H | Br | H | " | |
| 90 | $OCH_3$ | H | Cl | H | H | H | $OCF_3$ | H | " | |

Die N-Benzoyl-,N'-(1,1-difluor-phenylmethyl)-phenylharnstoffe der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz-und Veterinärsektor als Schädlingsbekämpfungsmittel eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella -(Getreidemotte), Phthorimaea operculella -(Kartoffelmotte), Capua reticulana (Apfelschalenwickler), Sparganothis pilleriana -(Springwurm), Cacoecia murinana -(Tannentriebwickler), Tortrix viridana -(Eichenwickler), Clysia ambiguella (Heu-und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella -(Wachsmotte), Malacosoma neustria (Ringelspinner), Dendrolimus pini (Kiefernspinner), Thaumatopoea pityocampa (Pinienprozessionsspinner), Phalera bucephala -(Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum -(Wintersaateule), Agrotis ypsilon (Ypsiloneule), Barathra brassicae (Kohleule), Cirphis unipuncta -(Heerwurm), Prodenia litura (Baumwollraupe), Laphygma exigua (Rüben-Heerwurm), Panolis flammea (Forleule), Earias insulana (Baumwollkapselwurm), Plusia gamma -(Gammaeule), Alabama argillacea (Baumwollblattwurm), Lymantria dispar -(Schwammspinner), Lymantria monacha (Nonne), Pieris brassicae (Kohlweißling), Aporia crataegi -(Baumweißling);

aus der Ordnung der Käfer (Coleoptera) beispielsweise Blitophaga undata (Schwarzer Rübenaaskäfer), Melanotus communis -(Drahtwurm), Limonius californicus (Drahtwurm), Agriotes lineatus (Saatschnellkäfer), Agriotes obscurus (Humusschnellkäfer), Agrilus sinuatus -(Birnbaum-Prachtkäfer), Meligethes aeneus -(Rapsglanzkäfer), Atomaria linearis -(Moosknopfkäfer), Epilachna varivestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis -(Brachkäfer), Crioceris asparagi -(Spargelhähnchen), Lema melanopus (Getreidehähnchen), Leptinotarsa decemlineata -(Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis -(Linsenkäfer), Bruchus rufimanus -(Pferdebohnenkäfer), Bruchus pisorum -(Erbsenkäfer), Sitona lineatus (Liniierter Blattrandkäfer), Otiorrhynchus sulcatus (Gefurchter Lappenrüßler), Otiorrhynchus ovatus -(Erdbeerwurzelrüßler), Hylobies abietis (Großer Brauner Rüsselkäfer), Byctiscus betulae -(Rebenstecher), Anthonomus pomorum (Apfelblütenstecher), Anthonomus grandis -(Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise Lycoria pectoralis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea -(Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata -(Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ocis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida - (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfaltenlaus), Brachycaudus cardui (Große Pflaumenblattlaus), Brevicoryne brassicae (Kohlblattlaus), Phorodon humuli - (Hopfenblattlaus), Rhopalomyzus ascalonicus - (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae - (Tannentrieblaus), Dreyfusia piceae (Weißtannenstammlaus), Adelges laricis (Rote Fichtengallenlaus), Viteus vitifolii (Reblaus);

aus der Ordnung der Termiten (Isoptera) beispielsweise Reticulitermes lucifugus, Calotermes flavicollis, Leucotermes flavipes, Termes natalensis;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus - (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melano plus femurrubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Zur Klasse der Arachnoidea gehören Spinnentiere (Acarina) beispielsweise Ixodes ricinus - (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa.

Zur Klasse der Nemathelminthes zählen beispielsweise Wurzelgallennematoden, z.B. Meloidogyne incognita, Meloidogyne hapla, Meloidogyne javanica, Zysten bildende Nematoden, z.B. Heterodera rostochiensis, Heterodera schachtii, Heterodera avenae, Heterodera glycines, Heterodera trifolii, Stock-und Blattälchen, z.B. Ditylenchus dipsaci, Ditylenchus destructor, Pratylenchus neglectus, Pratylenchus penetrans, Partylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-thylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier-oder Emulgiermittel in Wasser homogenisiert werden. Es können aber auch aus wirksamer Substanz Netz-, Haft-, Dispergier-oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali-und Erdalkalisalze der Dibutylnaphthalinsulfonsäure, Laurylethersulfat, Fettalkoholsulfate, fettsaure Alkali-und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu-und Stäubemittel können durch Mischen oder gemeinsames Vermahlen der wirksamen Substanzen mit einem festen Trägerstoff hergestellt werden.

Beispiele für Formulierungen sind:

I. 5 Gewichtsteile der Verbindung Nr. 1 werden mit 95 Gewichtsteilen feinteiligem Kaolin innig vermischt. Man erhält auf diese Weise ein Stäubemittel, das 3 Gew.% des Wirkstoffs enthält.

II. 30 Gewichtsteile der Verbindung Nr. 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gewichtsteile der Verbindung Nr. 4 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung Nr. 13 werden in einer Mischung • gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

V. 80 Gewichtsteile der Verbindung Nr. 19 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium-und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz-und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und/ %. Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,2 bis 10, vorzugsweise 0,5 bis 2,0 kg/ha.

Zu den Wirkstoffen können Öle verschiedenen Typs, Herbizide, Fungizide, andere Schädlingsbekämpfungsmittel, Bakteri-zide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibrom-ethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-

dimethylcarbamat, 2-Methyl-2-(methylthio)-propionaldehyd-0-(methylcarbamoyl)-oxim, S-Methyl-N-(methylcarbamoyl)-oxy]-thio -acetimidat, Methyl-N',N'-dimethyl-N-[(methylcarbamoyl)oxy]-1-thiooxamidat, N-(2-Methyl-chlor-phenyl)-N',N'-dimethylformamidin, Tetrachlorthiophen, 1-(2,6-Difluorbenzoyl)-3-(4-chlor-phenyl)-harnstoff, 0,0-Dimethyl-0-(p-nitrophenyl)-phosphorthioat, 0,0-Diethyl-0-(p-nitrophenyl)-phosphorthioat, 0-Ethyl-0-(p-nitrophenyl)-phenyl-phosphonothioat, 0,0-Di-methyl-0-(3-methyl-4-nitrophenyl)-phosphorthioat, 0,0-Diethyl-0--(2,4-dichlorphenyl)-phosphorthioat, 0-Ethyl-0-(2,4-dichlorphenyl)-phenyl-phosphonothioat, 0,0-Dimethyl-0-(2,4,5-trichlorphenyl)-phosphorthioat, 0-Ethyl-0-(2,4,5-trichlorphenyl)-ethyl-phosphonothioat, 0,0-Dimethyl-0-(4-brom-2,5-dichlorphenyl)-phosphorthioat, 0,0-Dimethyl-0-(2,5-dichlor-4-jod-phenyl)-phosphorthioat, 0,0-Dimethyl-0-(3-methyl-4-methylthiophenyl)-phosphorthioat, 0-Ethyl-0-(3-methyl-4-methylthiophenyl)-isopropyl-phosphoramidat, 0,0-Diethyl-0-[p-methylsulfinyl-phenyl],-phosphorthioat, 0-Ethyl-S-phenyl-ethylphosphonodithioat, 0,0-Diethyl-[2-chlor-1-(2,4-dichlorphenyl)-vinyl]-phosphat, 0,0-Dimethyl-[-2-chlor-1-(2,4,5-trichlorphenyl)],-vinyl-phosphat, 0,0-Dimethyl-S-(1'-phenyl)-ethylacetat-phosphordithioat, Bis-(dimethylamino)-fluorphosphinoxid, Octamethylpyrophosphoramid, 0,0,0,0-Tetraethyldithio-pyrophosphat, S-Chlormethyl-0,0-diethyl-phosphordithioat, 0-Ethyl-S,S-dipropyl-phosphordithioat, 0,0-Dimethyl-0-2,2--dichlorvinyl-phosphat, 0,0-Dimethyl-1,2-dibrom-2,2-di-chlorethylphosphat, 0,0-Dimethyl-2,2,2-trichlor-1-hydroxy-ethylphosphonat, 0,0-Dimethyl-S-[1,2-biscarbethoxy-ethyl-(1)]-phosphordithioat, 0,0-Dimethyl-0-(1-methyl-2-carbmethoxy-vinyl)-phosphat, 0,0-Dimethyl-S-(N-methyl-carbamoyl-methyl)-phosphordithioat, 0,0-Dimethyl-S-(N-methyl-carbamoyl-methyl)-phosphorthioat, 0,0-Dimethyl-S-(N-methoxyethyl-carbamoyl-methyl)-phosphordithioat, 0,0-Dimethyl-S-(N-formyl-N-methyl-carbamoylmethyl-phosphordithioat, 0,0-Dimethyl-0-[1-methyl-2-(methyl-carbamoyl)-vinyl]-phosphat, 0,0-Dimethyl-0-[(1-methyl-2-dimethylcarbamoyl)-vinyl]-phosphat, 0,0-Dimethyl-0-[(1-methyl-2-chlor-2-diethylcarbamoyl)-vinyl]-phosphat, 0,0-Diethyl-S-(ethyl-thio-methyl)-phosphordithioat, 0,0-Diethyl-S-[-(p-chlorphenylthio)-methyl]-phosphordithioat, 0,0-Dimethyl-S-(2--ethylthioethyl)-phosphorthioat, 0,0-Dimethyl-S-(2-ethylthioethyl)-phosphordithioat, 0,0-Dimethyl-S-(2-ethylsulfinyl-ethyl)-phosphorthioat, 0,0-Diethyl-S-(2-ethylthio-ethyl)-phosphordithioat, 0,0-Diethyl-S-(2-ethylsulfinyl-ethyl)-phosphorthioat, 0,0-Diethyl-thiophosphoryliminophenyl-acetonitril, 0,0-Diethyl-S-(2-chlor-1-phthalimidoethyl)-phosphordithioat, 0,0-Diethyl-S-[6-chlor-benzoxazolon-(2)-yl(3)]-methyldithiophosphat, 0,0-Dimethyl-S-[2-methoxy-1,3,4-thiadiazol-5-[4H]-onyl-(4)-methyl]-phosphordithioat, 0,0-Diethyl-0-[3,5,6-trichlor-pyridyl-(2)]-phosphorthioat, 0,0-Diethyl-0-(2-pyrazinyl)-phosphorthioat, 0,0-Diethyl-0-[2-isopropyl-4-methyl-pyrimidinyl(6)]-phosphorthioat, 0,0-Diethyl-0-[2-(diethylamino)-6-methyl-4pyrimidinyl]-thionophosphat, 0,0-Dimethyl -S-(4-oxo-1,2,3-benzotriazin-3-[4H]-yl-methyl)-phosphordithioat, 0,0-Dimethyl-S-[(4,6-diamino-1,3,5-triazin-2-yl)-methyl]-phosphordithioat, 0,0-Diethyl-(1-phenyl-1,2,4-triazol-3-yl)-thionophosphat, 0,S-Dimethyl-phosphor-amido-thioat, 0,S-Dimethyl-N-acetyl-phosphoramidothioat, alpha-Hexachlorcyclohexan, 1,1-Di-(p-methoxyphenyl)-2,2,2-trichlorethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis, trans-chrysanthemat, 5-Benzyl-furyl-(3)-methyl-DL-cis, trans-chrysanthemat, 3-Phenoxybenzyl (±)-cis,trans-2,2-dimethyl-3-(2,2-dichlorvinyl)-cyclopropancarboxylat, alpha-Cyano-3-phenoxybenzyl(±)-cis, trans-2,2-dimethyl-3-(2,2--dichlorvinyl)-cyclopropancarboxylat, (s)-alpha-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,trans-chrysanthemat, 2-Methyl-5-(2-propinyl)--3-furylmethyl-chrysanthemat, (alpha-Cyano-3-phenoxybenzyl)-alpha-isopropyl-4-chlorphenylacetat.

Anwendungsbeispiele

In den folgenden Beispielen wurden die erfindungsgemäßen Verbindungen hinsichtlich ihrer Wirkung auf Schädlinge gegen die nachstehenden Verbindungen des Standes der Technik verglichen.

I 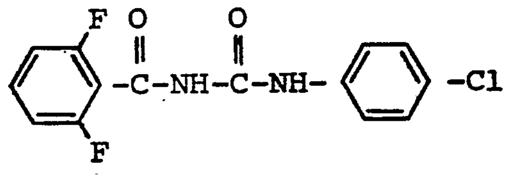 Diflubenzuron (ein Handelsprodukt);

II 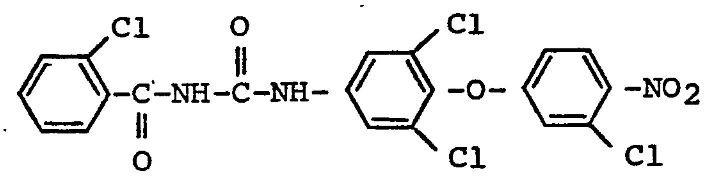

und

III 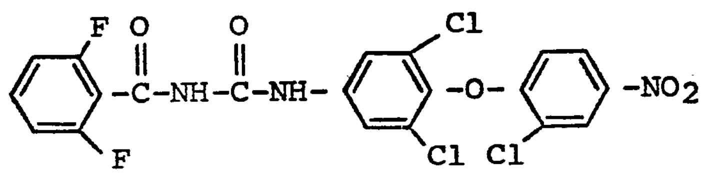

bekannt aus der DE-0S 25 04 984.

Die Konzentrationen, bei denen die Vergleichsverbindungen 100 % Mortalität bzw. Hemmung bewirken, sind die jeweiligen Minimalkonzentrationen. Bei jeder Konzentration wurden mindestens 2 Becher angesetzt.

Beispiel A

Entwicklungshemmung bei Prodenia litura (Prüfung auf behandeltem Nährboden)

100 g des Standard-Nährbodens für Prodenia wurden in 250 ml-Becher gefüllt und warm mit der wäßrigen Wirkstoffaufbereitung sorgfältig gemischt.

Nach Erkalten belegte man jedes Gefäß mit 10 Raupen im 3. Larvenstadium 1,5 -1,8 cm) und lagerte sie bei 23°C. Beurteilt wurde die Verpuppung und der Schlupf der Falter.

Bei diesem Test erzielten die Verbindungen 26 und 28 eine wenigstens 25-mal höhere Aktivität als die wirksamste Vergleichsverbindung (Verbindung I).

Beispiel B

Zuchtversuch mit Maiszünsler-Larven (Ostrinia nubilalis)

Die Zucht erfolgt auf einem Nährboden folgender Zusammensetzung:

515 g Maismehl

130 g Weizenkeime

137 g Bierhefe

18 g Ascorbinsäure

10 g Cellulosepulver

5 g Nipagin

20 g Wessons Salz

20 ml Vitaminlösung

80 g Agar

3100 ml Wasser

Jeder 250 ml-Becher wurde mit 100 ml Nährsubstrat und 10 Raupen im 3. Larvenstadium belegt und die Verpuppung und der Schlupf der Falter beurteilt. Die Aufwandmengen an Wirkstoff waren im Fall der Verbindungen 4 und 28 im 25-mal geringer als bei der Vergleichsverbindung I.

Beispiel C

Zuchtversuch mit Baumwollwanzen (Dysdercus intermedius)

Baumwollwanzen. Dysdercus intermedius wurden im 4. Larvenstadium in Petrischalen mit einem Durchmesser von 10 cm dem Wirkstoffbelag der Testsubstanz im Milligrammbereich für 24 Stunden ausgesetzt.

Die Überlebenden züchtete man in 1 l-Gläsern auf feuchtem Quarzsand, der vorher mit der Wirkstofflösung im ppm-Bereich versetzt wurde bis zum Schlüpfen der $F_1$-Generation.

Dabei entsprachen in der Behandlung

2,5 mg/Schale 25 ppm im Sand

1,0 mg/Schale 10 ppm im Sand

0,5 mg/Schale 5 ppm im Sand

usw.

Beurteilt wird Mortalität und Vermehrung. Die Versuche zeigten, daß eine Aufwandmenge von 2 ppm im Sand (0,2 mg pro Schale) eine quantitative Mortalität ergab.

Beispiel D

Chemosterilisation bei Stubenfliegen (Musca domestica)

Ca. 100 schlüpfreife Puppen von Stubenfliegen wurden in Folienbehälter mit 8,5 l Volumen gesetzt. Sie erhielten hier 2 g Futter, bestehend aus

6 Teilen Kristallzucker

6 Teilen Trockenmilch

1 Teil Eipulver

dem zunächst 20 mg des Wirkstoffes in organischer Lösung zugefügt wurden (= 1 Gew.%).

Wasser wurde in ausreichender Menge auf Zellstoff in 100 ml Kunststoffbechern geboten. Die Tiere wurden ca. 8 Tage in diesen Käfigen bei 22°C im Tag-und Nachtrhytmus gehalten.

Am 8. Tag gab man ein Eiablagegefäß mit in Magermilch getränktem Zellstoff in die Käfige. Hier erfolgte innerhalb der nächsten 24 Stunden die Eiablage.

Beim Einstellen der Eiablagegefäße wurde die Fraßgiftwirkung anhand der toten Fliegen beurteilt.

Nach 3 -4 Tagen wurde die Entwicklung der Eier auf der Milchwatte beurteilt.

Das Ergebnis des Versuches zeigte, daß Verbindung 4 eine 3-mal so hohe Wirkung aufwies als die Vergleichsverbindung I. Im Fall der Verbindung 28 war die Wirkung mehr als das 100-fache der Verbindung I.

Beispiel E

Zuchtversuch mit Moskito-Larven (Aedes aegypti)

200 ml Leitungswasser in 250 ml Plastikbechern wurden mit der Wirkstoffaufbereitung versetzt. Darauf belegte man jeden Becher mit ca. 50 schlüpfreifen Eiern von Aedes aegypti, die auf der Papierunterlage haften, welche wir bei der Eiablage boten.

Die Versuchstemperatur betrug 25°C. Beurteilt wurde die Entwicklung und der Schlupf der Imagines, wobei eine unbehandelte Kontrolle als Maßstab diente.

Während der Versuchsdauer wurde nach Bedarf mit einem handelsüblichen Zierfischfutter gefüttert.

Die Ergebnisse des Versuches zeigten, daß die Verbindung 2 um das 10-fache wirksamer war, als die Vergleichsverbindung II. Die Wirkstärke der Verbindungen 4,26 und 28 war um mehr als das 20-fache größer als bei der Vergleichsverbindung II.

## Ansprüche

1. N-Benzoyl-,N'-(1,1-difluor-phenylmethyl)-phenylharnstoffe der Formeln Ia bzw. Ib.

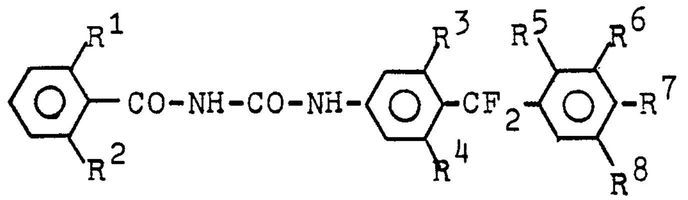

(Ia) -

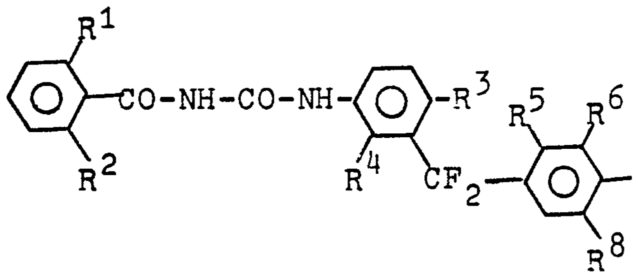

(Ib),

in denen bedeutet

$R^1$ Fluor, Chlor, Brom, Methyl oder Methoxy

$R^2$ Fluor, Chlor oder Wasserstoff

$R^3$ und $R^4$ gleich oder verschiedene Substituenten, nämlich Wasserstoff, Chlor, Brom, Fluor oder Trifluormethyl

$R^5$ bis $R^8$ Wasserstoff oder mindestens einen Substituenten, nämlich Fluor, Chlor, Brom, Alkyl mit 1 bis 4 C-Atomen, das mit Fluor und/oder Chlor substituiert sein kann, Alkoxy mit 1 bis 4 C-Atomen, das mit Fluor und/oder Chlor substituiert ist.

2. Verfahren zur Herstellung von N-Benzoyl-,N'-(1,1-difluor-phenylmethyl)-phenylharnstoffen - (Ia,b) gemäß Anspruch 1, dadurch gekennzeichnet, daß man ein entsprechendes (1,1-Difluor-phenylmethyl)-phenylamin II

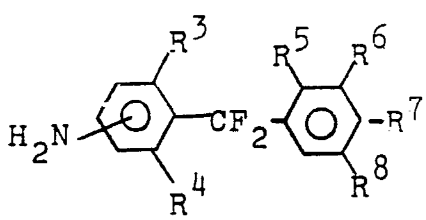

(II),

mit einem entsprechenden Benzoylisocyanat III

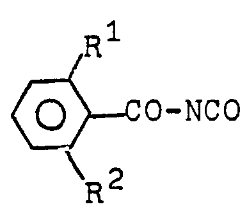

(III),

umsetzt.

3. Verfahren zur Herstellung von N-Benzoyl-,N'-(1,1-difluor-phenylmethyl)-phenylharnstoffen (Ia,b) gemäß Anspruch 1, dadurch gekennzeichnet, daß man ein entsprechendes Benzamid IV

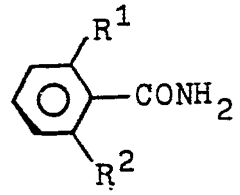 (IV),

mit einem entsprechenden Isocyanat V

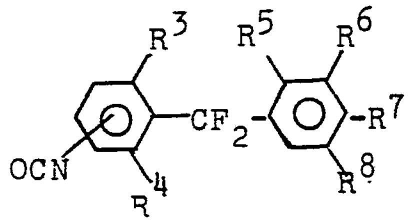 (V),

umsetzt.

4. Schädlingsbekämpfungsmittel, enthaltend einen Wirkstoff der Formel Ia oder Ib gemäß Anspruch 1.

5. Schädlingsbekämpfungsmittel, enthaltend einen festen oder flüssigen Trägerstoff und/oder mindestens einen weiteren Wirkstoff und einen Wirkstoff der Formel Ia bzw. Ib gemäß Anspruch 1.

6. Verwendung der N-Benzoyl-,N'-(1,1-difluor-phenylmethyl)-phenylharnstoffe der Formel Ia bzw. Ib gemäß Anspruch 1 bzw. von Mitteln gemäß Anspruch 4 oder 5 zur Bekämpfung von Schädlingen, insbesondere Insekten und Akariden.

7. Verfahren zur Bekämpfung von Schädlingen, insbesondere Insekten und Akariden, dadurch gekennzeichnet, daß man eine wirksame Menge eines N-Benzoyl-,N'-(1,1-difluor-phenylmethyl)-phenylharnstoffs der Formel Ia bzw. Ib gemäß Anspruch 1 auf die Schädlinge und/oder deren Lebensraum einwirken läßt.

Patentansprüche für den Vertragsstaat : AT

1. Verfahren zur Herstellung von N-Benzoyl-,N'-(1,1-difluor-phenylmethyl)-phenylharnstoffen dar allgemeinen Formeln Ia und Ib

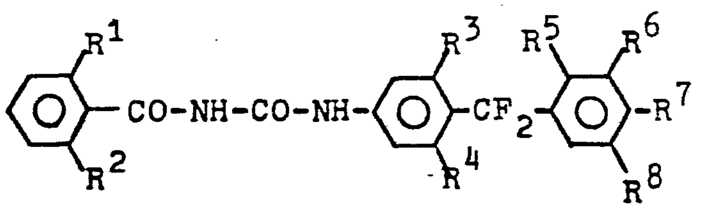 (Ia)

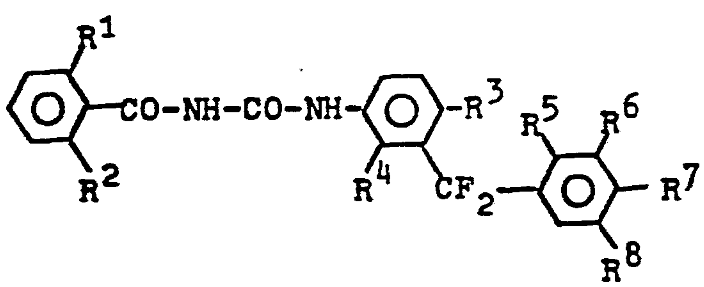 (Ib),

in denen die Substituenten folgende Bedeutung haben;

$R^1$ Fluor, Chlor, Brom, Methyl oder Methoxy

$R^2$ Fluor, Chlor oder Wasserstoff

$R^3$ und $R^4$ gleich oder verschiedene Substituenten, nämlich Wasserstoff, Chlor, Brom, Fluor oder Trifluormethyl

$R^5$ bis $R^8$ Wasserstoff oder mindestens einen Substituenten, nämlich Fluor, Chlor, Brom, Alkyl mit 1

bis 4 C-Atomen, das mit Fluor und/oder Chlor substituiert sein kann, Alkoxy mit 1 bis 4 C-Atomen, das mit Fluor und/oder Chlor substituiert ist, dadurch gekennzeichnet, daß man ein entsprechendes (1-1-Difluor-phenylmethyl)-phenylamin II

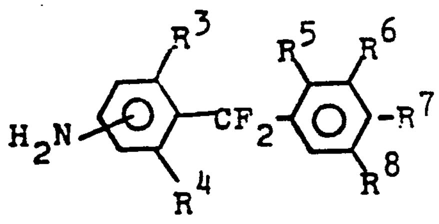 (II),

mit einem entsprechenen Benzoylisocyanat III

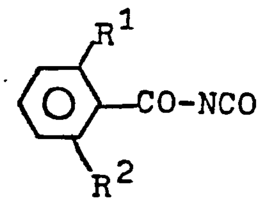 (III),

umsetzt.

2. Verfahren zur Herstellung von N-Benzoyl-,N'-(1,1-difluor-phenylmethyl)-phenylharnstoffen (Ia,b) gemäß Anspruch 1, dadurch gekennzeichnet, daß man ein entsprechendes Benzamid IV

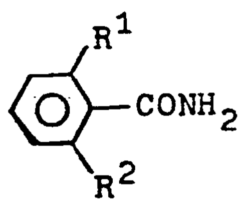 (IV),

mit einem entsprechenden Isocyanat V

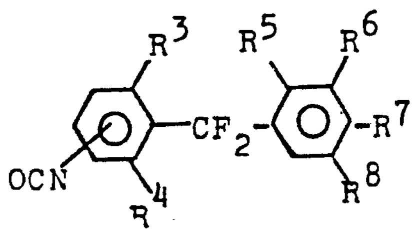 (V),

umsetzt.

3. Schädlingsbekämpfungsmittel, enthaltend einen Wirkstoff der Formel Ia oder Ib gemäß den Ansprüchen 1 und 2.

4. Schädlingsbekämpfungsmittel, enthaltend einen festen oder flüssigen Trägerstoff und/oder mindestens einen weiteren Wirkstoff und einen Wirkstoff der Formel Ia bzw. Ib gemäß den Ansprüchen 1 und 2.

5. Verwendung der N-Benzoyl-,N'-(1,1-difluorphenylmethyl)-phenylharnstoffe der Formel Ia bzw. Ib gemäß den Asnprüchen 1 und 2 bzw. von Mitteln gemäß Anspruch 3 oder 4 zur Bekämpfung von Schädlingen, insbesondere Insekten und Akariden.

6. Verfahren zur Bekämpfung von Schädlingen, insbesondere Insekten und Akariden, dadurch gekennzeichnet, daß man eine wirksame Menge eines N-Benzoyl-,N'-(1,1-difluor-phenylmethy)-phenylharnstoffes der Formel Ia bzw. Ib gemäß den Ansprüchen 1 und 2 auf die Schädlinge und/oder deren Lebenstraum einwirken läßt.

Europäisches Patentamt

**EUROPÄISCHER RECHERCHENBERICHT**

Nummer der Anmeldung

EP 86107329.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | EP - A2 - 0 159 587 (BASF)<br>* Patentansprüche 1-7 *<br>-- | 1-7 | C 07 C 127/22<br>A 01 N 47/34 |
| A | EP - A2 - 0 123 302 (MONTEDISON S.P.A)<br>* Patentansprüche 1,7,10,11 *<br>-- | 1,2, 4-7 | |
| A | WO - A1 - 85/ 01 047 (FMC CORPORATION)<br>* Patentansprüche 1,9,10 *<br>-- | 1,4,7 | |
| A | GB - A - 2 106 499 (DOW CHEMICAL COMPANY)<br>* Patentanspruch1*<br>-- | 1,3 | RECHERCHIERTE SACHGEBIETE (Int. Cl.4) |
| P,A | US - A - 4 536 341 (RAYMOND H. RIGTERNIK et aL)<br>* Spalten 1,2 *<br>-- | 1,4,6 | C 07 C 127/00 |
| D,A | DE - A - 2 123 236 (N.V.PHILIPS')<br>* Patentansprüche 1,2,44,75,76, 77,120,153 *<br>---- | 1-7 | |

Der vorliegende Recherchenbericht wurde für alle Patentansprüche erstellt.

| Recherchenort | Abschlußdatum der Recherche | Prüfer |
|---|---|---|
| WIEN | 22-07-1986 | REIF |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82